# EUROPEAN PATENT SPECIFICATION

(11) **EP 3 678 540 B1**
(45) Date of publication and mention of the grant of the patent: **19.02.2025**
(21) Application number: 18854149.4
(22) Date of filing: 05.09.2018
(51) Int. Cl.: A61B 5/024, A61B 5/11, A61B 5/1455

(54) **METHOD OF OBTAINING BIOMETRIC INFORMATION BASED ON WEARING STATE AND ELECTRONIC DEVICE THEREOF**
VERFAHREN ZUR GEWINNUNG BIOMETRISCHER INFORMATIONEN BASIEREND AUF EINEM VERSCHLEISSZUSTAND UND ELEKTRONISCHE VORRICHTUNG DAFÜR
PROCÉDÉ D'OBTENTION D'INFORMATIONS BIOMÉTRIQUES SUR LA BASE D'UN ÉTAT DE PORT ET DISPOSITIF ÉLECTRONIQUE ASSOCIÉ

(30) Priority: 06.09.2017 KR 20170114055
(43) Date of publication of application: 15.07.2020
(73) Proprietor: Samsung Electronics Co., Ltd., Suwon-si, Gyeonggi-do 16677 (KR)
(72) Inventor: KIM, Taehyeon, Gyeonggi-do 16677 (KR); LEE, Jeongsu, Gyeonggi-do 16677 (KR); LIM, Daehyeong, Gyeonggi-do 16677 (KR); CHO, Minhyun, Gyeonggi-do 16677 (KR); SEO, Jinwoo, Gyeonggi-do 16677 (KR); PARK, Jeongmin, Gyeonggi-do 16677 (KR)
(74) Representative: Nederlandsch Octrooibureau
(86) International application number: PCT/KR2018/010374
(87) International publication number: WO 2019/050277

(56) References cited:
- WO-A1-2017/115361
- JP-A- 2004 261 366
- US-A1- 2011 092 780
- US-A1- 2015 216 484
- US-A1- 2016 058 374
- US-A1- 2016 199 002
- US-A1- 2017 112 398
- US-A1- 2017 215 778
- US-A1- 2017 238 819

## Description

### Technical Field

The disclosure relates to an electronic device. More particularly, the disclosure relates to an electronic device that can obtain a user's biometric information upon the device being worn at a user's body.

### Background Art

Recently, with the development of mobile communication technology and process technology, electronic devices have been changed to a form that can freely access to a wired or wireless network while being easily carried, and the functions that can be performed by the electronic device have been diversified. Further, the form of electronic devices has been diversified and, for example, an electronic device may be implemented into a form of a wearable device that a user can directly attach to a portion of the user's body for use. A wearable type electronic device may have, for example, a form of a wrist watch that can be attached to a user's wrist; and, in this case, the electronic device can perform various functions such as call and message transmission and reception, execution of various applications, and reproduction of multimedia contents in addition to a watch function.

Because a wearable type electronic device is directly attached to the user's body for use, the electronic device may be used for obtaining the user's biometric information using several types of biometric sensor. For example, the electronic device may measure the user's heart rate, stress, and blood oxygen saturation level SpO2 using a photoplethysmography (PPG) sensor. In this case, in order to use various output bands, the PPG sensor may obtain biometric information using light of various bands such as green, red, and infra-red (IR), and the electronic device may have at least two light emitting elements and/or light receiving elements.

When biometric information is obtained according to an output band (e.g., green, red, IR), there are merits and demerits in a light emitting element provided in the electronic device. For example, in a green band of a low wavelength, accurate measurement is difficult because of low permeability of the skin. Meanwhile, an IR or red band of a high wavelength has high skin permeability, but it is sensitive to a motion; thus, when a motion of the electronic device is large, accurate measurement is difficult in the IR or red band. Even if a conventional electronic device supports various output ranges of light emitting elements, the conventional electronic device does not consider a use state thereof; thus, it is difficult to measure accurately biological information, and there is a problem of high power consumption by use of a plurality of light emitting elements.

The above information is presented as background information only to assist with an understanding of the disclosure. No determination has been made, and no assertion is made, as to whether any of the above might be applicable as prior art with regard to the disclosure.

The document WO2017/115361 A1, disclosed on 6 July 2017, describes wearable devices, systems and methods for noninvasive monitoring and analyzing of physiological measurements.

The document US2016/0058374 A1, disclosed on 3 March 2016, describes a wearable electronic apparatus detects movement of a human body wearing the apparatus.

The document US2016/199002 A1, disclosed on 14 July 2016, describes a wearable electronic device for monitoring physiological measurements and detecting a distance between the device and the user.

### Disclosure of Invention

### Technical Problem

Aspects of the disclosure are to address at least the above-mentioned problems and/or disadvantages and to provide at least the advantages described below.

An aspect of the disclosure is to provide an electronic device that can measure more accurately biological information in consideration of a use state thereof.

Additional aspects will be set forth in part in the description which follows and, in part, will be apparent from the description, or may be learned by practice of the presented embodiments.

### Solution to Problem

The invention is set out in the appended set of claims.

Other aspects, advantages, and salient features of the disclosure will become apparent to those skilled in the art from the following detailed description, which, taken in conjunction with the annexed drawings, discloses various embodiments of the disclosure.

### Advantageous Effects of Invention

An aspect of the disclosure can provide an electronic device that can measure more accurately biological information in consideration of a use state thereof.

### Brief Description of Drawings

The above and other aspects, features, and advantages of certain embodiments of the disclosure will be more apparent from the following description taken in conjunction with the accompanying drawings, in which:
FIG. 1 is a block diagram illustrating a configuration of an electronic device in a network environment for obtaining biometric information based on a wearing state according to various embodiments of the disclosure;
FIGS. 2A and 2B are diagrams illustrating an outer shape of an electronic device according to various embodiments of the disclosure;
FIG. 3 is a diagram illustrating a principle of an optical sensor module according to various embodiments of the disclosure;
FIG. 4 is a block diagram illustrating a configuration of an electronic device according to various embodiments of the disclosure;
FIG. 5 is a block diagram illustrating a configuration of an electronic device according to various embodiments of the disclosure;
FIG. 6 is a flowchart illustrating a method of obtaining biometric information of an electronic device according to various embodiments of the disclosure;
FIG. 7 is a flowchart illustrating an operation of detecting whether an electronic device is being worn according to various embodiments of the disclosure;
FIG. 8 is a flowchart illustrating an operation of determining a wearing state of an electronic device according to various embodiments of the disclosure;
FIGS. 9A, 9B, 9C, and 9D are diagrams illustrating a wearing state of an electronic device and graphs illustrating an optical signal measured in each wearing state according to various embodiments of the disclosure;
FIG. 10 is a flowchart illustrating a motion state determining an operation of an electronic device according to various embodiments of the disclosure;
FIG. 11 is a table illustrating a biological information measuring function that may be performed according to a state of an electronic device according to various embodiments of the disclosure; and
FIG. 12 is a flowchart illustrating a method of obtaining biometric information with an electronic device according to various embodiments of the disclosure.

Throughout the drawings, like reference numerals will be understood to refer to like parts, components, and structures.

### Best Mode for Carrying out the Invention

The following description with reference to the accompanying drawings is provided to assist in a comprehensive understanding of various embodiments of the disclosure as defined by the claims. It includes various specific details to assist in that understanding but these are to be regarded as merely exemplary. Accordingly, those of ordinary skill in the art will recognize that various changes and modifications of the various embodiments described herein can be made without departing from the scope of the disclosure. In addition, descriptions of well-known functions and constructions may be omitted for clarity and conciseness.

The terms and words used in the following description and claims are not limited to the bibliographical meanings, but, are merely used by the inventor to enable a clear and consistent understanding of the disclosure. Accordingly, it should be apparent to those skilled in the art that the following description of various embodiments of the disclosure is provided for illustration purpose only and not for the purpose of limiting the disclosure as defined by the appended claims.

It is to be understood that the singular forms "a," "an," and "the" include plural referents unless the context clearly dictates otherwise. Thus, for example, reference to "a component surface" includes reference to one or more of such surfaces.

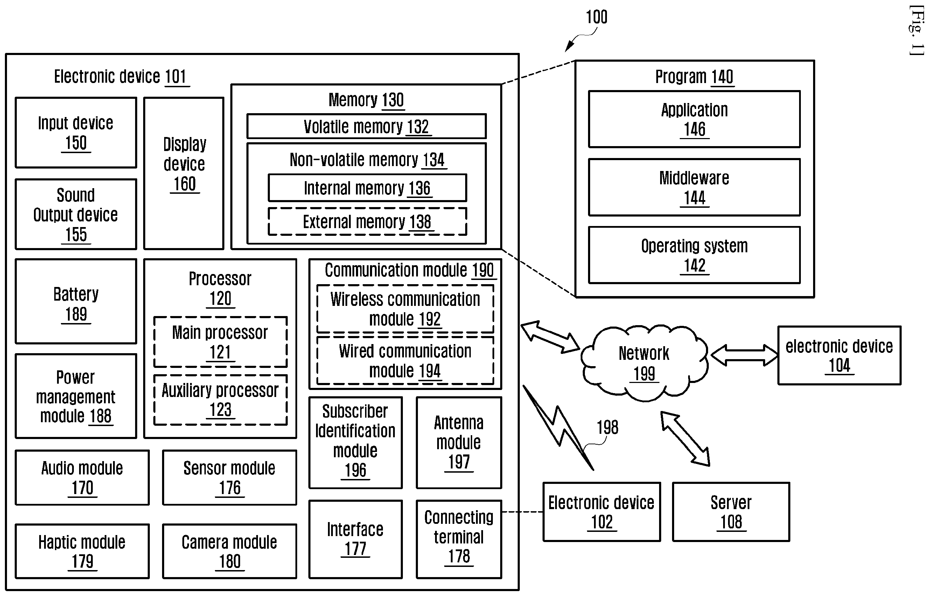

FIG. 1 is a block diagram illustrating an electronic device 101 in a network environment 100 according to various embodiments of the disclosure.

Referring to FIG. 1, the electronic device 101 in the network environment 100 may communicate with an electronic device 102 via a first network 198 (e.g., a short-range wireless communication network), or an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input device 150, a sound output device 155, a display device 160, an audio module 170, a sensor module 176, an interface 177, a haptic module 179, a camera module 180, a power management module 188, a battery 189, a communication module 190, a subscriber identification module (SIM) 196, or an antenna module 197. In some embodiments, at least one (e.g., the display device 160 or the camera module 180) of the components may be omitted from the electronic device 101, or one or more other components may be added in the electronic device 101. In some embodiments, some of the components may be implemented as single integrated circuitry. For example, the sensor module 176 (e.g., a fingerprint sensor, an iris sensor, or an illuminance sensor) may be implemented as embedded in the display device 160 (e.g., a display).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may load a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), and an auxiliary processor 123 (e.g., a graphics processing unit (GPU), an image signal processor (ISP), a sensor hub processor, or a communication processor (CP)) that is operable independently from, or in conjunction with, the main processor 121. Additionally or alternatively, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display device 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input device 150 may receive a command or data to be used by other component (e.g., the processor 120) of the electronic device 101, from the outside (e.g., a user) of the electronic device 101. The input device 150 may include, for example, a microphone, a mouse, or a keyboard.

The sound output device 155 may output sound signals to the outside of the electronic device 101. The sound output device 155 may include, for example, a speaker or a receiver. The speaker may be used for general purposes, such as playing multimedia or playing record, and the receiver may be used for incoming calls. According to an embodiment, the receiver may be implemented as separate from, or as part of the speaker.

The display device 160 may visually provide information to the outside (e.g., a user) of the electronic device 101. The display device 160 may include, for example, a display, a hologram device, or a projector and control circuitry to control a corresponding one of the display, hologram device, and projector. According to an embodiment, the display device 160 may include touch circuitry adapted to detect a touch, or sensor circuitry (e.g., a pressure sensor) adapted to measure the intensity of force incurred by the touch.

The audio module 170 may convert a sound into an electrical signal and vice versa. According to an embodiment, the audio module 170 may obtain the sound via the input device 150, or output the sound via the sound output device 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as BluetoothTM, wireless-fidelity (Wi-Fi) direct, or infrared data association (IrDA)) or the second network 199 (e.g., a long-range communication network, such as a cellular network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The antenna module 197 may transmit or receive a signal or power to or from the outside (e.g., the external electronic device) of the electronic device 101. According to an embodiment, the antenna module 197 may include one or more antennas, and, therefrom, at least one antenna appropriate for a communication scheme used in the communication network, such as the first network 198 or the second network 199, may be selected, for example, by the communication module 190 (e.g., the wireless communication module 192). The signal or the power may then be transmitted or received between the communication module 190 and the external electronic device via the selected at least one antenna.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 and 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, or client-server computing technology may be used, for example.

The electronic device according to various embodiments may be one of various types of electronic devices. The electronic devices may include, for example, a portable communication device (e.g., a smart phone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., Play StoreTM), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

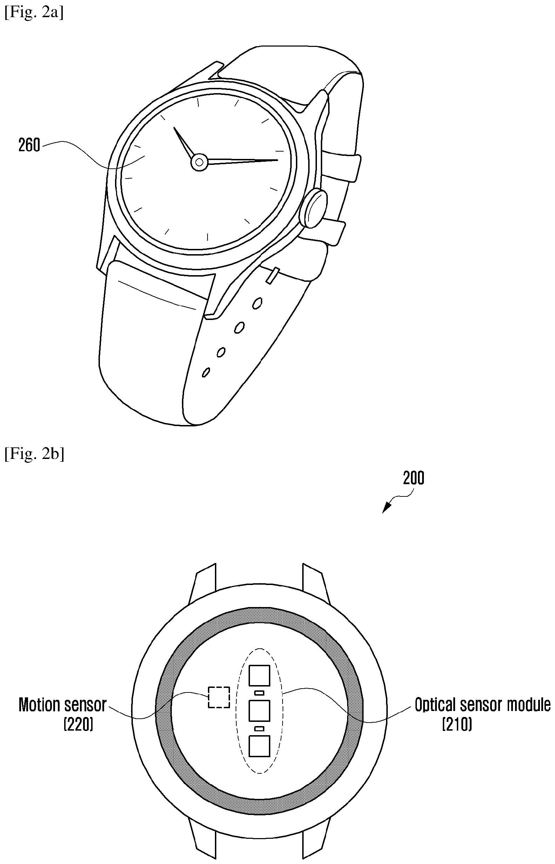

FIGS. 2A and 2B are diagrams illustrating an outer shape of an electronic device according to various embodiments of the disclosure.

Referring to FIG. 2A, an electronic device 200 may be a wrist wearable device (e.g., watch, bracelet type, band type, bangle type). However, the disclosure is not limited thereto and various kinds of electronic devices that can obtain the user's biological information when the user approaches by having an optical sensor module (e.g., a photoplethysmography (PPG) sensor) mounted may correspond to the electronic device 200 of the disclosure. For example, the electronic device 200 according to various embodiments may be implemented into a smart phone, a head-mounted device (e.g., a virtual reality (VR) device, augmented reality (AR) device, mixed reality (MR) device, glasses type device), body attachment device (e.g., health patch, digital tattoo), clothing type device (e.g., smart clothing, glove, footwear), and band type device (e.g., wrist/arm band, smart ring) having a biometric sensor.

The electronic device 200 may include a body and a strap. At a front surface (e.g., a surface exposed to the outside when being worn by the user) of the body, a display 260 is provided to display various application screens such as a message and a call as well as time information. The user of the electronic device 200 may wear the electronic device 200 at a part of the user's body (e.g., wrist) using a strap.

Referring to FIG. 2B illustrates a rear surface (e.g., a surface that contacts with the user's body when being worn by the user) of the body of the electronic device 200. According to various embodiments, the electronic device 200 includes an optical sensor module 210, and a light emitting element and a light receiving element of the optical sensor module 210 may be exposed from the rear surface of the body to the outside to output light to an external object and detect reflected light of the output light.

According to various embodiments, the optical sensor module 210 may include a PPG sensor. The PPG sensor may detect, in the light receiving element (e.g., a photodiode), reflected light reflected by an external object (e.g., the user's body) among light output from a light emitting element (e.g., LED) and measure biometric information such as the user's heart rate, stress, and blood oxygen saturation level SpO2 based on reflected light detected by the light receiving element.

According to various embodiments, the optical sensor module 210 may include at least one light emitting element and one light receiving element. For example, as shown in FIG. 2B, the optical sensor module 210 may include three light emitting elements and two light receiving elements, but the number and/or a disposition form of the light emitting elements and the light receiving elements are/is not limited to that of FIG. 2B. According to an embodiment, in the optical sensor module 210, the light emitting elements and the light receiving elements may be disposed in line to detect effectively a motion of the electronic device, and a light receiving element of excellent signal detection may be selected.

The light emitting element may include an LED that can output light of a visible light range (e.g., green, red) and/or an infrared band. For example, the light emitting element may output green light having a wavelength of 520 to 565 nm, red light having a wavelength of 660 nm, and/or infra-red (IR) light having a wavelength of 880 to 940 nm. When green light is used, the green light may be strong while in motion, but the green light may have low skin permeability; and when red light or IR light is used, the red light or IR light may have high skin permeability, but the red light or IR light may have weak signal strength and be sensitive while in motion.

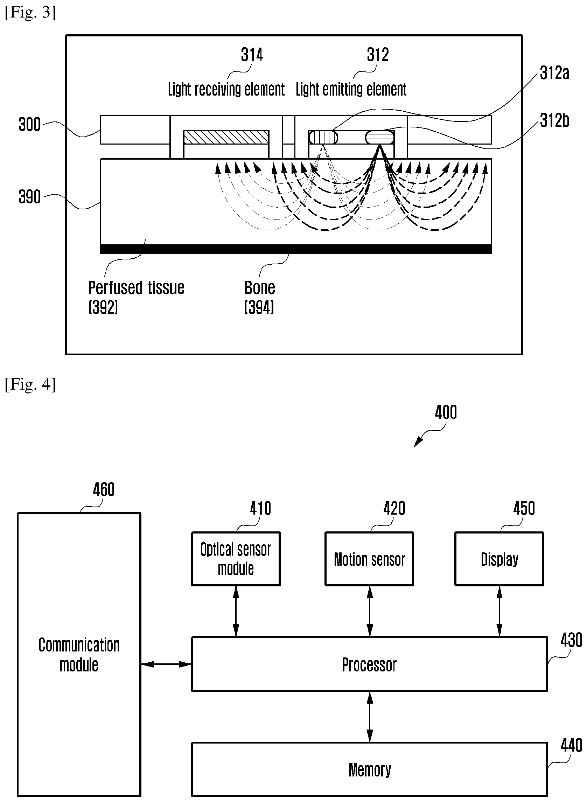

According to various embodiments, each light emitting element may output light of a fixed wavelength band (e.g., the first light emitting element outputs green light, and the second light emitting element outputs light of an IR band) or each light emitting element may output light of various wavelengths. The light receiving element may include a photodiode that can detect an optical signal to convert and output the optical signal to an electric signal, and each light receiving element may detect reflected light of light output from a corresponding one light emitting element or may detect reflected light of light output from all light emitting elements. A principle of obtaining the user's biometric information using the optical sensor module 210 will be described in detail with reference to FIG. 3.

According to various embodiments, the electronic device 200 may further include a motion sensor 220. The motion sensor 220 may be implemented into various types of sensors that can detect a motion of the electronic device 200 such as a gyro sensor and an acceleration sensor. The electronic device 200 may determine a motion level thereof based on a sensing signal of the motion sensor 220 and determine a motion state (e.g., a normal state, sleep state, exercise state) thereof.

According to various embodiments of the disclosure, in order to obtain biometric information in consideration of a use state (e.g., a wearing state and/or a motion state) of the electronic device 200, the electronic device 200 may selectively drive a light emitting element and/or a light receiving element. Therefore, the electronic device 200 can provide various differentiated pieces of biometric information to the user and achieve effects such as increased accuracy of a measuring signal, enhancement of current consumption, and removal of inconvenience upon measuring the user's biological information.

FIG. 3 is a diagram illustrating a principle of an optical sensor module according to various embodiments of the disclosure.

Referring to FIG. 3 illustrates a principle of detecting, through a light receiving element 314, reflected light reflected by collision with an external object 390 (e.g., a user wrist) among light output from a light emitting element 312 (e.g., a first light emitting element 312a and a second light emitting element 312b) in a state in which a user wears an electronic device 300. FIG. 3 illustrates two light emitting elements 312a and 312b and one light receiving element 314; but, as described above, the electronic device 300 may include a plurality of light emitting elements 312 and/or light receiving elements 314, and the number of the plurality of light emitting elements 312 and/or light receiving elements 314 is not limited.

The light emitting elements 312 may output light of a specific wavelength band (e.g., green, red, IR) according to a control signal of the processor (e.g., the processor 120 of FIG. 1 and a processor 430 of FIG. 4). Light output from the light emitting element 312 may be reflected through a perfused tissue 392 and/or a bone 394, and a property of an optical signal received in the light receiving element 314 may be changed according to the user's body situation. For example, when blood flowing through a blood vessel of a user's wrist increases, the blood vessel expands; thus, an amount of reflected light detected by the light receiving element 314 may decrease. In this way, the electronic device 300 may measure biometric information such as the user's heart rate, stress, and blood oxygen saturation level SpO2 according to an attribute of reflected light detected by the light receiving element 314.

FIG. 4 is a block diagram illustrating a configuration of an electronic device according to various embodiments of the disclosure.

Referring to FIG. 4, an electronic device 400 may include an optical sensor module 410, a motion sensor 420, a processor 430 (e.g., at least one processor), a memory 440 (e.g., a storage), a display 450, and a communication module 460 (e.g., a transceiver), and even if at least some of the illustrated elements are omitted or substituted, as needed, various embodiments of the disclosure may be implemented. The electronic device 400 may be a wearable device as described with reference to FIGS. 2A and 2B, but the disclosure is not limited thereto and may be implemented into a smart phone, head-mounted device (e.g., VR device, AR device, MR device, glasses-type device), body attachment device (e.g., health patch, digital tattoo), clothing type device (e.g., smart clothing, gloves, footwear), and band type device (e.g., wrist/arm band, smart ring) that can obtain the user's biometric information when the user approaches by being mounted in the optical sensor module 410.

The optical sensor module 410 may include a light emitting module and a light receiving module, the light emitting module may include a plurality of light emitting elements (e.g., the first light emitting element 312a and the second light emitting element 312b of FIG. 3), and the light receiving module may include a plurality of light receiving elements (e.g., a first light receiving element and a second light receiving element). Hereinafter, it is described that a light emitting module includes a first light emitting element and a second light emitting element, but the number of the light emitting elements included in the light emitting module is not limited thereto.

As described with reference to FIG. 2B above, the light emitting element and the light receiving element may be exposed from a rear surface of the body of the electronic device 400 to the outside to contact (or adjacent to) with the user's body when the user wears the electronic device 400. The optical sensor module 410 may be electrically connected to the processor 430, an output wavelength and a light emitting element to be driven according to a control signal of the processor 430 may be determined, and an optical signal detected by the light receiving element may be converted to an electrical signal to be provided to the processor 430.

According to various embodiments, the first light emitting element of the light emitting module may have a first attribute and the second light emitting element thereof may have a second attribute, wherein the first attribute and the second attribute may be an attribute related to output intensity and/or an output wavelength of the light emitting element. According to various embodiments, at least one of output intensity and an output wavelength of the first light emitting element and the second light emitting element may have different attributes.

The motion sensor 420 may be implemented into various type sensors that may detect a motion of the electronic device 400, such as a gyro sensor and an acceleration sensor. The motion sensor 420 may be electrically connected to the processor 430 to provide a motion signal generated according to motion detection of the electronic device 400 to the processor 430.

The memory 440 may include a volatile memory 440 (e.g., the volatile memory 132 of FIG. 1) and/or a non-volatile memory 440 (e.g., the non-volatile memory 134 of FIG. 1) and may be electrically connected to the processor 430. The memory 440 may store various instructions that may be performed in the processor 430. Such instructions may include a control command such as arithmetic and logic operations, data movement, and an input and output that may be recognized by the control circuit and may be defined on a framework stored at the memory 440. Further, the memory 440 may store at least some of the program 140 of FIG. 1.

According to various embodiments, the processor 430 may perform an operation or data processing related to the control and/or communication of each element of the electronic device 400 and include at least some of the configurations of/or functions of the processor 120 of FIG. 1. According to other embodiments, the processor 430 may be included in a sensor hub that performs a sensor control and detection of a sensing signal of several sensors (e.g., the optical sensor module 410, the motion sensors 420) provided in the electronic device 400. The processor 430 may be electrically connected to internal components of the electronic device 400, such as the optical sensor module 410, the motion sensor 420, the memory 440, the display 450, and the communication module 460.

Operations and data processing functions that the processor 430 may implement within the electronic device 400 are not limited, and hereinafter various embodiments will be described in which the processor 430 determines a use state (e.g., a wearing state and a motion state) of the electronic device 400 and controls the optical sensor module 410 based on the use state to obtain biometric information. Operations of the processor 430 to be described later may be performed by loading the foregoing instructions stored at the memory 440.

The display 450 displays an image and may be implemented into a liquid crystal display (LCD), light emitting diode (LED) display, and organic light emitting diode (OLED). The display 450 may include at least some of the configurations and/or functions of the display device 160 of FIG. 1 and may be disposed in a form of the display 260 of FIG. 2A. When obtaining biometric information, the processor 430 may display visual information generated by the biometric information and a necessary visual notification related to obtaining of the biometric information on the display 450.

The communication module 460 transmits and receives data to and from various external electronic devices 400 and may include at least some of the configurations and/or functions of the communication module 190 of FIG. 1.

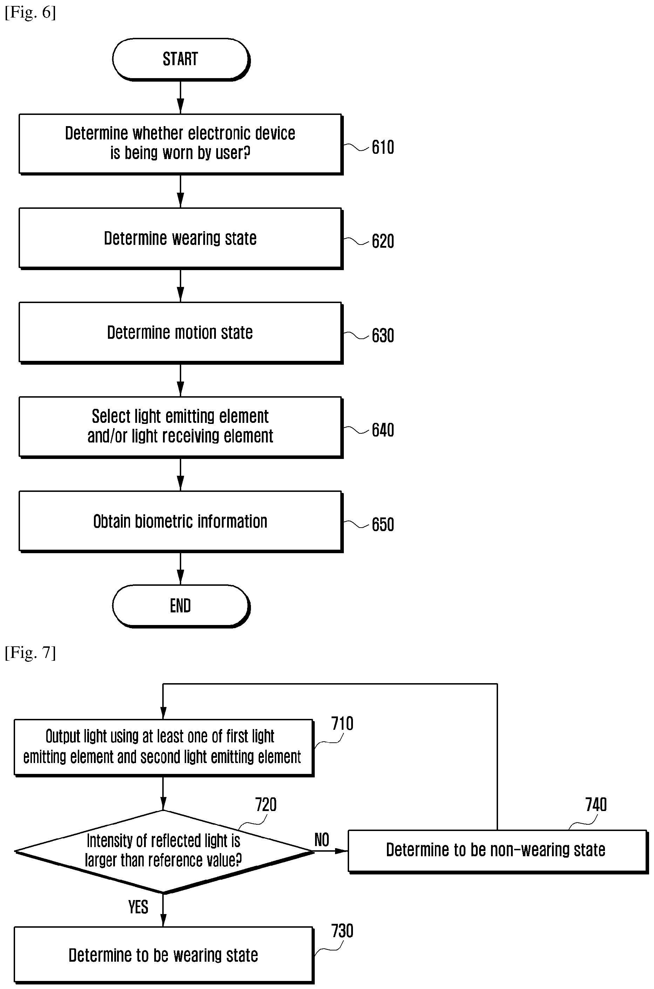

According to various embodiments, the processor 430 may obtain biometric information such as a heart rate, stress, and blood oxygen saturation level SpO2 of an external object (e.g., a user's body) using the optical sensor module 410. For obtaining desired biometric information, the processor 430 may detect whether the electronic device 400 is being worn and determine a wearing state (e.g., tight or loose) and/or a motion state (e.g., a normal state, sleep state, exercise state). The processor 430 may determine a light emitting element and/or a light receiving element to use for obtaining biological information based on the determined wearing state and/or motion state. A specific operation of the processor 430 will be described in detail with reference to FIGS. 6 to 12.

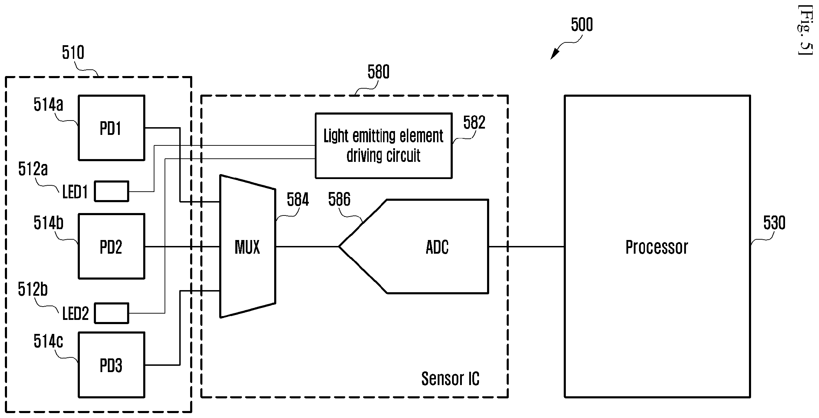

FIG. 5 is a block diagram illustrating a configuration of an electronic device according to various embodiments of the disclosure.

Referring to FIG. 5 illustrates a circuit structure for controlling each of light emitting elements (or modules) 512a and 512b and/or light receiving elements (or modules) 514a, 514b, and 514c of an optical sensor module such as a biometric sensor module 510 (e.g., the optical sensor module 410 of FIG. 4).

As shown in FIG. 5, the optical sensor module or biometric sensor module 510 may include a plurality of light emitting elements (e.g., a first light emitting element 512a and a second light emitting element 512b) and a plurality of light receiving elements or modules (e.g., a first light receiving element 514a, a second light receiving element 514b, and a third light receiving element 514c). The plurality of light emitting elements 512a and 512b and the plurality of light receiving elements 514a, 514b, and 514c may be disposed in line, as described with reference to FIG. 2B, but the disclosure is not limited thereto.

A sensor circuit 580 is electrically provided between a processor 530 and the optical sensor module 510 and drives each of the light emitting elements 512a and 512b according to a control signal of the processor 530 and provides signals received from each of the light receiving element 514a, 514b, and 514c to the processor 530. The sensor circuit 580 may include a light emitting element driving circuit 582, multiplexer (MUX) 584, and analog to digital converter (ADC) 586.

The light emitting element driving circuit 582 may drive the first light emitting element 512a and/or the second light emitting element 512b according to a control signal of the processor 530. According to an embodiment, the first light emitting element 512a and the second light emitting element 512b may have different attributes in output intensity and output wavelength; and, in this case, the processor 530 may determine at least one of the first light emitting element 512a and the second light emitting element 512b to use for measurement of biological information to output a control signal to the light emitting element driving circuit 582, and the light emitting element driving circuit 582 may drive the first light emitting element 512a and/or the second light emitting element 512b based on the received control signal. According to another embodiment, the first light emitting element 512a and the second light emitting element 512b each may have various output intensities and output wavelengths. In this case, the control signal may include information about a light emitting element to be driven and output intensity and an output wavelength of light to output from each light emitting element.

The first light receiving element to the third light receiving element 514a, 514b, and 514c obtain an optical signal (e.g., analog electric signals corresponding to a detected light amount) and output the optical signal to the MUX 584, and the MUX 584 adds signals transmitted from each of the light receiving elements 514a, 514b, and 514c to a single channel to output the signals to the ADC 586. According to another embodiment, the MUX 584 may output a signal transmitted from each of the light receiving elements 514a, 514b, and 514c to the ADC 586 through a separate channel. The ADC 586 may convert the received optical signal to a digital signal to output the digital signal to the processor 530.

An electronic device 500 according to various embodiments includes a biometric sensor module 510 including light emitting modules including a first light emitting element 512a having a first attribute and a second light emitting element 512b having a second attribute and light receiving elements 514a, 514b, and 514c disposed adjacent to the light emitting elements 512a and 512b; and a processor 530, wherein the processor 530 is configured to determine a distance between the biometric sensor module 510 and an external object based on at least reflected light reflected by collision with the external object among light output from the light emitting elements 512a and 512b, to select at least one of the first light emitting element 512a and the second light emitting element 512b based on at least the distance, and to obtain biometric information about the external object using the selected at least one light emitting element.

According to various embodiments, at least one of output intensity and an output wavelength of the first light emitting element 512a and the second light emitting element 512b may have different attributes.

According to various embodiments, the light receiving elements 514a, 514b, and 514c may include a first light receiving element 514a and a second light receiving element 514b, and the processor 530 may be configured to select at least one light receiving element configured to detect the reflected light based on at least the distance.

According to various embodiments, the processor 530 may be configured to control at least one of the first light emitting element 512a and the second light emitting element 512b to output light and to control the first light emitting element 512a and the second light emitting element 512b to output light when an intensity of reflected light corresponding to the output light is larger than a designated value.

According to various embodiments, the processor 530 may be configured to determine a close contact level between the biometric sensor module 510 and the external object to be a first close contact level or a second close contact level based on at least the determined distance between the biometric sensor module 510 and the external object and to select at least one light emitting element configured to obtain biological information based on at least the determined close contact level.

According to various embodiments, the processor 530 may be configured to drive any one of the first light emitting element 512a and the second light emitting element 512b to obtain the biometric information, when the close contact level is determined to be a first close contact level; and to drive at least one of the first light emitting element 512a and the second light emitting element 512b to obtain the biological information, when the close contact level is determined to be a second close contact level.

According to various embodiments, the electronic device 500 may further include a motion sensor 420, wherein the processor 530 may be configured to determine a state of the electronic device 500 to be one of a first state and a second state using the motion sensor 420 and to drive at least one of the first light emitting element 512a and the second light emitting element 512b based on the first state and the second state to obtain biometric information about the external object.

According to various embodiments, when a state of the electronic device 500 is a second state, the processor 530 may be configured to drive the second light emitting element 512b designated to correspond to the second state.

An electronic device 500 according to various embodiments includes a biometric sensor module 510 including a plurality of light emitting elements 512a and 512b and a plurality of light receiving elements 514a, 514b, and 514c; and a processor 530 electrically connected to the biometric sensor module 510, wherein the processor 530 is configured to control the plurality of light emitting elements 512a and 512b to output light, to drive the plurality of light receiving elements 514a, 514b, and 514c to obtain an optical signal detected by the plurality of light receiving element 514a, 514b, and 514c, to determine a distance between each of the light emitting elements 512a and 512b and an external object based on the optical signal, to determine a wearing state of the electronic device 500 based on the distance, to determine at least one of the plurality of light emitting elements 512a and 512b to use for obtaining biometric information and an output wavelength of the at least one light emitting element based on the determined wearing state, to determine at least one of the plurality of light receiving elements 514a, 514b, and 514c configured to use for obtaining biological information based on the determined wearing state, and to obtain biometric information about the external object based on an optical signal detected by the determined at least one light receiving element.

According to various embodiments, the processor 530 may be configured to obtain motion information of the electronic device 500; to determine a motion state of the electronic device 500 based on the obtained motion information; to determine at least one of the plurality of light emitting elements 512a and 512b and an output wavelength of the at least one light emitting element based on the determined motion state; and to determine at least one of the plurality of light receiving elements 514a, 514b, and 514c based on the determined motion state.

According to various embodiments, the processor 530 may be configured to determine, if an average value of a distance between each of the light emitting elements 512a and 512b and the external object is smaller than a designated value, the wearing state to be the first state and determine, if an average value of a distance between each of the light emitting elements 512a and 512b and the external object is larger than or equal to a designated value, the wearing state to be the second state and to select at least two of the plurality of light emitting elements 512a and 512b and to determine an output wavelength of the selected at least two light emitting elements to different values, when the wearing state is determined to be the first state, and to select one of the plurality of light emitting elements 512a and 512b when the wearing state is determined to be the second state.

According to various embodiments, the processor 530 may be configured to obtain at least one type biometric information corresponding to the wearing state and the motion state.

FIG. 6 is a flowchart illustrating a method of obtaining biometric information of an electronic device according to various embodiments of the disclosure.

Referring to FIG. 6, the illustrated method may be performed by a processor (e.g., the processor 430 of FIG. 4, the processor 530 of FIG. 5) of the electronic device, and hereinafter a description of the foregoing technical characteristics will be omitted.

The processor may determine whether the electronic device is worn by the user at operation 610. The processor may output light through at least one predetermined light emitting element among a plurality of light emitting elements (e.g., the first light emitting element 512a, the second light emitting element 512b of FIG. 5) of optical sensor modules (e.g., the optical sensor module 510 of FIG. 5) and determine whether the electronic device is being worn through an intensity of reflected light detected by a light receiving element (e.g., the first light receiving element 514a, the second light receiving element 514b, and the third light receiving element 514c of FIG. 5). A detailed operation of operation 610 will be described in detail with reference to FIG. 7.

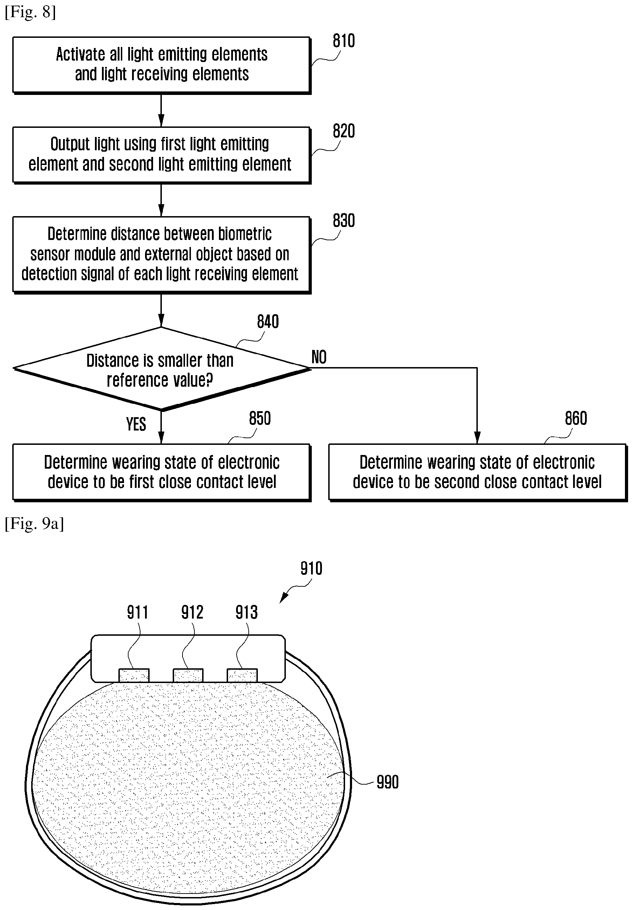

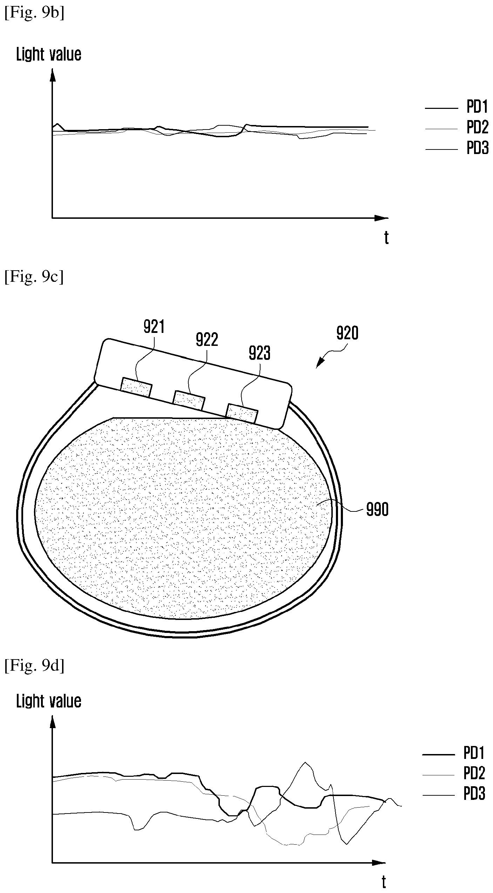

If the electronic device is being worn by the user, the processor may determine a wearing state (or a close contact level) of the electronic device at operation 620. The processor may determine whether the electronic device is appropriately (or tightly) worn or inappropriately (or loosely) worn using two or more light emitting elements or all light emitting elements. A detailed operation of operation 620 will be described in detail with reference to FIGS. 8, 9A and 9B.

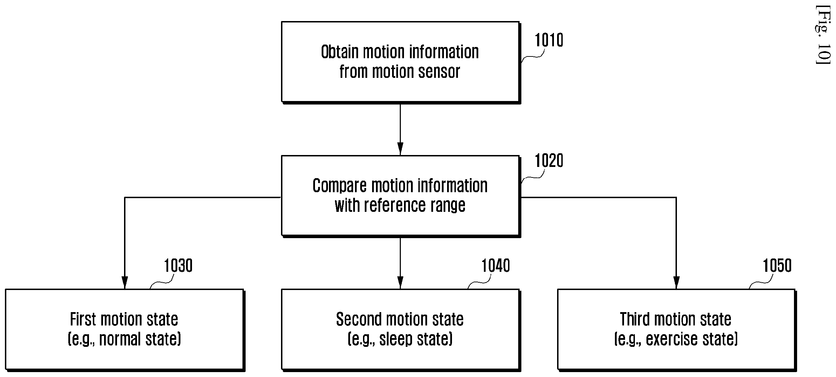

The processor may determine a motion state of the electronic device at operation 630. The processor may classify a wearing state of the electronic device into a normal state, sleep state, and exercise state based on the sensing result of the motion sensor (e.g., the motion sensor 420 of FIG. 4). It is not necessary to perform operation 630 after operation 620, and operation 630 may be performed at least partially simultaneously with operation 620 or may be performed before operation 620. Operation 630 will be described in detail with reference to FIG 10.

The processor may select, at operation 640, a light emitting element and/or a light receiving element to use for obtaining biometric information based on at least the determined wearing state and/or motion state of the electronic device. According to various embodiments, the processor may extract information mapped to the determined wearing state and/or motion state from a table stored at the memory (e.g., the memory 440 of FIG. 4) and select a light emitting element and/or a light receiving element.

The processor may obtain biometric information (e.g., heart rate, stress, blood oxygen saturation level SpO2) at operation 650 using the light emitting element and the light receiving element determined at operation 640.

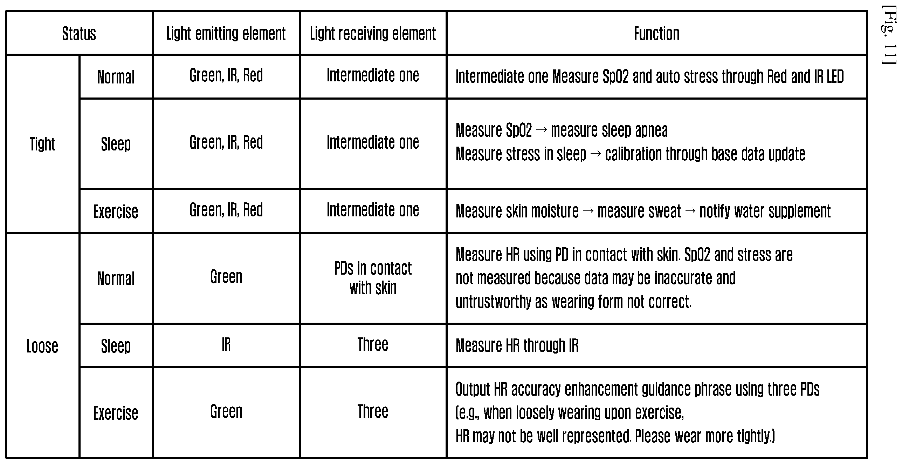

An embodiment of selecting the light emitting element and the light receiving element of operation 640 and an example of biometric information that may be obtained in operation 650 will be described in detail with reference to FIG. 11.

FIG. 7 is a flowchart illustrating an operation of detecting whether an electronic device is being worn according to various embodiments of the disclosure. The operations of FIG. 7 correspond to operation 610 of FIG. 6.

Referring to FIG. 7, in a state in which the electronic device is not worn, the processor (e.g., the processor 530 of FIG. 5) may control to output light using at least one of a predetermined first light emitting element (e.g., 512a of FIG. 5) and second light emitting element (e.g., 512b of FIG. 5) at operation 710. In a state in which the electronic device is not being worn, the processor may control a light emitting element to periodically output light and control to output light of an infra-red (IR) band that is invisible to the user.

The processor may compare an intensity of reflected light reflected by the user's body (390 of FIG. 3) among light output from the light emitting element with a designated value (e.g., a reference value) based on a signal received from the light receiving elements (e.g., 514a, 514b, and 514c of FIG. 5) at operation 720.

If the intensity of the reflected light is smaller than or equal to a designated value, the processor may determine at operation 740 that the electronic device is not being worn by the user; and the process continues to operation 710, and the processor may control to periodically output light using at least one of a predetermined first light emitting element and second light emitting element.

If an intensity of the reflected light is larger than a designated value, the processor may determine at operation 730 that the electronic device is being worn by the user. If the electronic device is being worn by the user, the process may enter operation 620 of FIG. 6 and the processor performs subsequent operations and controls the first light emitting element and the second light emitting element to output light.

FIG. 8 is a flowchart illustrating an operation of determining a wearing state of an electronic device according to various embodiments of the disclosure. FIGS. 9A to 9D illustrate a wearing state of the electronic device and are graphs of the optical signal measured in each wearing state according to various embodiments of the disclosure.

Referring to FIG. 8 correspond to operation 620 of FIG. 6, and hereinafter a process in which the processor determines a wearing state of the electronic device will be described with reference to FIG. 8 and FIGS. 9A to 9D. The following operations may be performed after it has been determined that the electronic device is being worn by the user through the process of FIG. 7.

The processor (e.g., the processor 530 of FIG. 5) may activate at least two light emitting elements (e.g., the first light emitting element and the second light emitting element) and at least one light receiving element of the optical sensor module (e.g., the optical sensor module 510 of FIG. 5) at operation 810. According to an embodiment, the processor may activate all light emitting elements and light receiving elements included in the optical sensor module.

The processor may output light using at least two light emitting elements (e.g., the first light emitting element and the second light emitting element) at operation 820.

The processor may receive a detection signal of each light receiving element (e.g., the first light receiving element to the third light receiving element) and determine, at operation 830, a distance between the biometric sensor module and the external object (e.g., the user's body) based on the detection signal.

FIGS. 9A and 9C are diagrams illustrating a state in which a user wears an electronic device having a first light receiving element to a third light receiving element.

FIG. 9A illustrates a state in which the user tightly wears the electronic device 910 and illustrates a state (e.g., a first close contact level) in which all light receiving elements 911, 912, and 913 come in close contact with a user's body 990. FIG. 9C illustrates a state in which the user loosely wears the electronic device 920, wherein a third light receiving element 923 may come in close contact with a user's body 990, but a first light receiving element 921 and a second light receiving element 922 may be somewhat separated from the user's body 990.

When the wearing state of the electronic device is a tight state as shown in FIG. 9A, a value of an optical signal detected by each of the light receiving elements 911, 912, and 913 is as shown in FIG. 9B. With reference to FIG. 9B, because distances between each of the light receiving elements 911, 912, and 913 and the user's body 990 are substantially the same, values of the optical signal detected by each of the light receiving elements 911, 912, and 913 sequentially have no large difference. According to various embodiments, as shown in FIG. 9A, when all of a plurality of light receiving elements 911, 912, and 913 contacts with the user's body 990, the processor may obtain a biological signal using only one light receiving element or all light receiving elements.

When a wearing state of the electronic device is a loose state as shown in FIG. 9C, a value of an optical signal detected by each of the light receiving elements 921, 922, and 923 is as shown in FIG. 9D. With reference to FIG. 9D, because there is a difference in a distance between each of the light receiving elements 921, 922, and 923 and the user's body 990, a value of the optical signals detected by each of the light receiving elements 921, 922, and 923 may show a large difference. According to various embodiments, as shown in FIG. 9C, when only some (e.g., the third light receiving element 923) of a plurality of light receiving elements contact with the user's body, the processor may obtain a biological signal using only the contact light receiving element (e.g., the third light receiving element 923). According to an embodiment, if the wearing state of the electronic device is a loose state, the processor may, for accurate measurement of biometric information, provide an alarm through the display (e.g., the display 450 of FIG. 4) for inducing the electronic device to be worn tightly by adjustment of a strap.

The processor may determine a wearing state of the electronic device to be a tight state of FIG. 9A or a loose state of FIG. 9C based on a correlation of an optical signal detected by each light receiving element. For example, the processor may determine a distance between the optical sensor module and the user's body based on a change rate of each optical signal value, a distribution value of an optical signal level unit, and a correlation between signals.

The processor may compare the determined distance between the biometric sensor module and the user's body with a designated value (e.g., a reference value) at operation 840.

If the distance is smaller than a designated value, the processor may determine, at operation 850, a wearing state of the electronic device to be a first close contact level (or a tight state or an appropriate wearing state).

If the distance is larger than or equal to a designated value, the processor may determine, at operation 860, a wearing state of the electronic device to be a second close contact level (or a loose state or an inappropriate wearing state).

According to an not-claimed embodiment, the processor may determine a wearing state of the electronic device based on at least a signal obtained from each light receiving element without determining a distance between the biometric sensor module and an external object. For example, if a correlation of the optical signal detected by each light receiving element belongs to a first range, the processor may determine a wearing state of the electronic device to be a first close contact level; and, if a correlation of the optical signal detected by each light receiving element belongs to a second range, the processor may determine a wearing state of the electronic device to be a second close contact level. In this case, the operation 830 to the operation 840 may be omitted.

FIG. 10 is a flowchart illustrating a motion state determining operation of an electronic device according to various embodiments of the disclosure. Operations of FIG. 10 may correspond to the operation 630 of FIG. 6 and may be performed at least partially simultaneously with the wearing state determination operation 620.

Referring to FIG. 10, the processor (e.g., the processor 430 of FIG. 4, the processor 530 of FIG. 5) may obtain motion information from the motion sensor (e.g., the motion sensor 420 of FIG. 4) at operation 1010. The motion sensor may be implemented into a gyro sensor, an acceleration sensor, or the like that may detect a motion state of the electronic device.

The processor may compare the obtained motion information (e.g., acceleration of the electronic device) with a designated range (e.g., a reference range) at operation 1020.

If a range to which the obtained motion information belongs is a smallest range, there is little motion in the electronic device; thus, the processor may determine, at operation 1040, a motion state of the electronic device to be a second motion state corresponding to a sleep state. According to an embodiment, the processor may determine that the user is in a lying state in additional consideration of an advancing direction of the electronic device; and, when a motion of the electronic device belongs to a designated range, the processor may determine a motion state of the electronic device to be a sleep state.

If a range to which the obtained motion information belongs is an intermediate range, the processor may determine, at operation 1030, a motion state of the electronic device to be a first motion state corresponding to a normal state in which the user wears the electronic device and generally lives his/her everyday life.

If a range to which the obtained motion information belongs is a largest range, the motion of the electronic device is in a largest state; thus, the processor may determine a motion state of the electronic device to be a third motion state corresponding to an exercise state at operation 1050.

FIG. 11 is a table illustrating a biological information measuring function that may be performed according to a state of an electronic device according to various embodiments of the disclosure.

Referring to FIG. 11, the processor (e.g., the processor 430 of FIG. 4, the processor 530 of FIG. 5) may determine a light emitting element and a light receiving element to use for obtaining biological information and a type of biometric information to obtain based on a wearing state of the electronic device determined using an optical sensor module (e.g., the optical sensor module 410 of FIG. 4, the optical sensor module 510 of FIG. 5) and a motion state determined using a motion sensor (e.g., the motion sensor 420 of FIG. 4). The processor may determine a light emitting element and a light receiving element and a kind of biological information to obtain according to a table stored at the memory (e.g., the memory 440 of FIG. 4), and FIG. 11 illustrates an example of the table.

In a state in which the user tightly wears the electronic device, although measurement accuracy of biological information through the optical sensor module increases, there may be deterioration in the user's wearing comfort. However, in a state in which the user loosely wears the electronic device, although the user's wearing comfort increases, the measurement accuracy may deteriorate when biological information is measured because of a motion effect.

According to various embodiments, when a wearing state of the electronic device is a tight state, an exposure possibility of light is low; thus, the processor may output light using a light emitting element (e.g., green, red, IR) of various bands, and because detection efficiency of the light receiving element is good, the processor may activate only one light receiving element. According to various embodiments, when a wearing state of the electronic device is a tight state, the processor may selectively drive, among the light emitting elements, any one light emitting element that can measure predetermined biological information (e.g., stress, blood oxygen saturation level) or biological information (e.g., stress, blood oxygen saturation level) selected by the user.

Further, when a wearing state of the electronic device is a loose state, the processor may output light using only a single light emitting element and activate at least one light receiving element.

With reference to the table of FIG. 11, if the wearing state is a loose state and the motion state is a normal state, the processor may use only a light emitting element of a green band among a plurality of light emitting elements. Because a green band, which is a relatively low wavelength band, is strong in a motion state, even when the wearing state is a loose state, accurate measurement of biological information is available.

In this case, by activating only a light receiving element (e.g., the third light receiving element 923 of FIG. 9C) that contacts with the user's body among a plurality of light receiving elements and by deactivating a light receiving element (e.g., the first light receiving element 921 and the second light receiving element 922 of FIG. 9C) separated from the user's body or exposed to external noise, measurement efficiency can be improved. Further, in a loose wearing state the processor may measure a heart rate in which a measured value is relatively simple; however, a stress and blood oxygen saturation level SpO2 may not be measured because a distance between the optical sensor module and the user's body is irregularly changed and it is difficult to accurately measure the data.

If the wearing state is a loose state and the motion state is a sleep state, the processor may output light through a light emitting element of an IR band among a plurality of light emitting elements and activate all light receiving elements. In this case, because visible light such as green or red is reflected from skin to be exposed to the outside, in order to avoid sleep disturbance by light, the processor may use only an invisible IR band.

If the wearing state is a loose state and the motion state is an exercise state, the processor may output light using only a light emitting element of a green band that is strong in a motion state in consideration of a user's motion. Further, the processor may activate all light receiving elements to measure effectively a heart rate. According to an embodiment, for measurement accuracy enhancement of biological information, the processor may output guidance through the display for wearing the electronic device more tightly.

If the wearing state is a tight state and the motion state is a normal state, the processor may output light using all light emitting elements. This is to obtain various biological information in addition to a heart rate using light of various bands. According to various embodiments, in this state, the processor may selectively drive any one light emitting element that can obtain predetermined biometric information or biometric information selected by the user. In this state, the processor may measure a stress and a blood oxygen saturation level SpO2 in real time. When a stress is measured in real time, the user's emotional state may be seen; thus, the processor may obtain more accurate biological information. Further, when the wearing state is a tight state, efficiency of the light receiving element is high; thus, the processor may activate only one light receiving element.

If the wearing state is a tight state and the motion state is a sleep state, the processor may output light using all light emitting elements and activate only one light receiving element. According to various embodiments, in this state, the processor may selectively drive any one light emitting element that can obtain predetermined biometric information or biometric information selected by the user. In a sleep state, the processor may determine sleep apnea through measurement of a blood oxygen saturation level. According to an embodiment, because a sleep state is the most stable state in daily life, the sleep state may be defined to a base value for measurement of a stress value at a normal state. In other words, by setting a value measured in a sleep state to a base value and by comparing measured biometric signals with the base value, the processor may measure stress values.

If the wearing state is a tight state and the motion state is an exercise state, the processor may additionally measure a skin moisture level. The processor may monitor in real time a dehydrated state during exercise through the skin moisture level and guide the user to drink water.

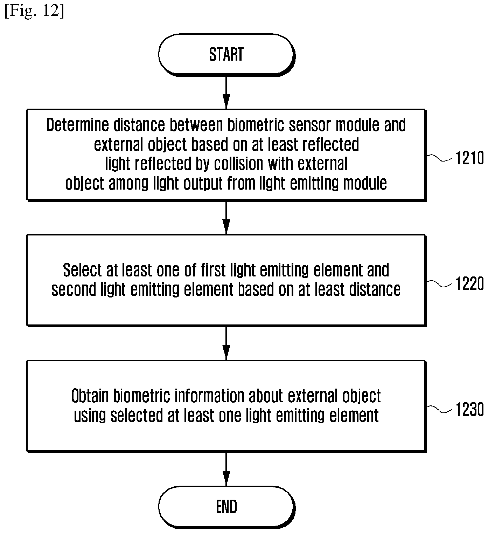

FIG. 12 is a flowchart illustrating a method of obtaining biometric information of an electronic device according to various embodiments of the disclosure.

Referring to FIG. 12, the illustrated method may be performed by the processor of the electronic device of FIG. 4, the electronic device includes an optical sensor module including a light emitting module and a light receiving module, and the light emitting module may include a first light emitting element and a second light emitting element having different attributes (e.g., output intensity and an output wavelength).

The processor may determine at operation 1210 a wearing state (e.g., tight or loose) of the electronic device based on at least reflected light reflected by collision with an external object among light output from the light emitting module. According to various embodiments, the processor may determine a distance between the biometric sensor module and the external object based on at least reflected light and determine a wearing state of the electronic device based on the determined distance. According to other not-claimed embodiments, the processor may determine a wearing state of the electronic device based on at least a signal obtained from each light receiving element without determining a distance between the biometric sensor module and the external object. For example, when a correlation of an optical signal detected by each light receiving element belongs to a first range, the processor may determine a wearing state of the electronic device to a first close contact level; and, when a correlation of an optical signal detected by each light receiving element belongs to a second range, the processor may determine a wearing state of the electronic device to a second close contact level.

The processor may select at operation 1220 at least one of the first light emitting element and the second light emitting element based on at least the wearing state. According to various embodiments, the processor may select a light emitting element based on a detection result of the motion sensor. An example has been described with reference to FIG. 11, in which the processor selects a light emitting element to use based on a motion state and a distance (or a wearing state of the electronic device) between the biometric sensor module and the external object.

The processor may obtain at operation 1230 biometric information about the external object using the selected at least one light emitting element. An example has been described with reference to FIG. 11, in which the processor obtains biometric information according to each use state of the electronic device.

In a method of obtaining biological information of an electronic device 500 according to various embodiments, the electronic device 500 includes a biometric sensor module 510 including light emitting modules including a first light emitting element 512a having a first attribute and a second light emitting element 512b having a second attribute and light receiving elements 514a, 514b, and 514c disposed adjacent to the light emitting elements 512a and 512b; and the method includes determining a distance between the biometric sensor module 510 and an external object based on at least reflected light reflected by collision with the external object among light output from the light emitting elements 512a and 512b, selecting at least one of the first light emitting element 512a and the second light emitting element 512b based on at least the distance, and obtaining biometric information about the external object using the selected at least one light emitting element.

According to various embodiments, at least one of output intensity and an output wavelength of the first light emitting element 512a and the second light emitting element 512b may have different attributes.

According to various embodiments, the light receiving elements 514a, 514b, and 514c may include a first light receiving element 514a and a second light receiving element 514b, and the method may further include selecting at least one light receiving element configured to detect the reflected light based on at least the distance.

According to various embodiments, the method may further include outputting light using at least one of the first light emitting element 512a and the second light emitting element 512b; and outputting, when intensity of reflected light corresponding to the output light is larger than a designated value, light by the first light emitting element 512a and the second light emitting element 512b.

According to various embodiments, determining a distance between the biometric sensor module 510 and an external object may include determining a close contact level between the biometric sensor module 510 and the external object to be a first close contact level or a second close contact level based on the determined distance between the biometric sensor module 510 and the external object, and selecting at least one of the first light emitting element and the second light emitting element may include selecting at least one light emitting element configured to obtain biological information according to the determined close contact level.

According to various embodiments, selecting at least one of the first light emitting element 512a and the second light emitting element 512b may include at least one of selecting, when the close contact level is determined to be a first close contact level, at least one of the first light emitting element 512a and the second light emitting element 512b; and driving, when the close contact level is determined to be a second close contact level, at least one of the first light emitting element and the second light emitting element to obtain the biometric information.

According to various embodiments, the method may further include determining a state of the electronic device 500 to be any one of the first state and the second state using a motion sensor 420, wherein selecting at least one of the first light emitting element and the second light emitting element may include driving at least one of the first light emitting element 512a and the second light emitting element 512b based on the first state or the second state.

According to various embodiments, selecting at least one of the first light emitting element and the second light emitting element may include selecting, when a state of the electronic device 500 is a second state, the second light emitting element 512b designated to correspond to the second state.

According to various embodiments of the disclosure, by determining a light emitting element and/or a light receiving element to use for obtaining biometric information according to a use state of an electronic device, accurate biological information corresponding to a situation can be measured.

While the disclosure has been shown and described with reference to various embodiments thereof, it will be understood by those skilled in the art that various changes in form and details may be made therein without departing from the scope of the invention as defined by the appended claims.

## Claims

1. An electronic device (500) comprising:
a biometric sensor module (510) including:
a light emitting module comprising a first light emitting element (512a) having a first attribute and a second light emitting element (512b) having a second attribute, and
a light receiving module including a plurality of light receiving elements (514a, 514b, 514c) disposed adjacent to the light emitting module;
a motion sensor configured to detect a motion of the electronic device (500); and
at least one processor (530) configured to:
determine a wearing state based on a distance between the biometric sensor module and an external object based on at least reflected light reflected by collision with the external object among light output from the first light emitting element and the second light emitting element of the light emitting module,
determine a motion state from one of a normal state, a sleep state, and an exercise state, based on motion information from the motion sensor,
select at least one of the first light emitting element or the second light emitting element based on the wearing state and the motion state, and
obtain biometric information about the external object using the selected light emitting element,
wherein if the distance is smaller than a designated value, the processor determines the wearing state to be a first close contact level corresponding to a tight state, outputs light using the first light emitting element and the second light emitting element of various bands and activates only one light receiving element of the plurality of light receiving elements,
wherein if the distance is larger than or equal to the designated value, the processor determines the wearing state to be a second close contact level corresponding to a loose state, selects one of the first light emitting element and the second light emitting element for outputting light based on the determined motion state, outputs light using the selected one of the first light emitting element of green band for the normal state or the exercise state and the second light emitting element of infra-red, IR, band for the sleep state and activates at least one light receiving element of the plurality of light receiving elements.

2. The electronic device of claim 1, wherein at least one of output intensity or an output wavelength of the first light emitting element and the second light emitting element have different attributes.

3. The electronic device of claim 1, wherein the at least one processor is further configured to:
control at least one of the first light emitting element and the second light emitting element to output light when an intensity of reflected light corresponding to the output light is smaller than or equal to a designated value; and
control the first light emitting element and the second light emitting element to output light when the intensity of the reflected light corresponding to the output light is larger than the designated value.

4. A method of obtaining biological information of an electronic device (500), wherein the electronic device (500) comprises a biometric sensor module comprising a light emitting module including a first light emitting element (512a) having a first attribute and a second light emitting element (512b) having a second attribute, and a light receiving module including a plurality of light receiving elements (514a, 514b, 514c) disposed adjacent to the light emitting module, the method being implemented by a processor of said electronic device and comprising:
determining (620) a wearing state based on a distance between the biometric sensor module and an external object based on at least reflected light reflected by collision with the external object among light output from the first light emitting element and the second light emitting element of light emitting module;
determining (630) a motion state from one of a normal state, a sleep state, and an exercise state, based on motion information from a motion sensor comprised in said electronic device;
selecting (640) at least one of the first light emitting element and the second light emitting element based on the wearing state and the motion state; and
obtaining (650) biometric information about the external object using the selected light emitting element,
wherein if the distance is smaller than a designated value, the method further comprising determining the wearing state to be a first close contact level corresponding to a tight state, outputting light using the first light emitting element and the second light emitting element of various bands and activating only one light receiving element of the plurality of light receiving elements,
wherein if the distance is larger than or equal to the designated value, the method further comprising determining the wearing state to be a second close contact level corresponding to a loose state, selecting one of the first light emitting element and the second light emitting element for outputting light based on the determined motion state, and outputting light using the selected one of the first light emitting element of green band for the normal state or the exercise state and the second light emitting element of infra-red, IR, band for the sleep state and activating at least one light receiving element of the plurality of light receiving elements.

5. The method of claim 4, wherein at least one of output intensity or an output wavelength of the first light emitting element and the second light emitting element have different attributes.

6. The method of claim 4, further comprising:
when an intensity of reflected light corresponding to the output light is smaller than or equal to a designated value, outputting light using at least one of the first light emitting element and the second light emitting element; and
when the intensity of the reflected light corresponding to the output light is larger than the designated value, outputting light by the first light emitting element and the second light emitting element.

## Patentansprüche

1. Elektronische Vorrichtung (500), die Folgendes umfasst:
ein biometrisches Sensormodul (510), das Folgendes enthält:
ein lichtemittierendes Modul, das ein erstes lichtemittierendes Element (512a) mit einem ersten Attribut und ein zweites lichtemittierendes Element (512b) mit einem zweiten Attribut umfasst, und
ein Lichtempfangsmodul, das eine Vielzahl von Lichtempfangselementen (514a, 514b, 514c) enthält, die angrenzend an das lichtemittierende Modul angeordnet sind;
einen Bewegungssensor, der so konfiguriert ist, dass er eine Bewegung der elektronischen Vorrichtung (500) erkennt; und
mindestens einen Prozessor (530), der zu Folgendem konfiguriert ist:
Bestimmen eines Tragezustands basierend auf einer Distanz zwischen dem biometrischen Sensormodul und einem externen Objekt basierend auf zumindest reflektiertem Licht, das durch eine Kollision mit dem externen Objekt unter Lichtausgabe von dem ersten lichtemittierenden Element und dem zweiten lichtemittierenden Element des lichtemittierenden Moduls reflektiert wird,
Bestimmen eines Bewegungszustands aus einem Normalzustand, einem Schlafzustand oder einem Übungszustand basierend auf Bewegungsinformationen von dem Bewegungssensor,
Auswählen des ersten lichtemittierenden Elements und/oder des zweiten lichtemittierenden Elements basierend auf dem Tragezustand und dem Bewegungszustand, und
Erhalten biometrischer Informationen über das externe Objekt unter Verwendung des ausgewählten lichtemittierenden Elements,
wobei, wenn die Distanz kleiner als ein festgelegter Wert ist, der Prozessor den Tragezustand als eine erste enge Kontaktebene bestimmt, die einem dichten Zustand entspricht, Licht unter Verwendung des ersten lichtemittierenden Elements und des zweiten lichtemittierenden Elements verschiedener Bänder ausgibt und nur ein Lichtempfangselement der Vielzahl von Lichtempfangselementen aktiviert,
wobei, wenn die Distanz größer als oder gleich dem festgelegten Wert ist, der Prozessor den Tragezustand als eine zweite enge Kontaktebene bestimmt, die einem lockeren Zustand entspricht, das erste lichtemittierende Element oder das zweite lichtemittierende Element zur Ausgabe von Licht basierend auf dem bestimmten Bewegungszustand auswählt, Licht unter Verwendung des ausgewählten ersten lichtemittierenden Elements des grünen Bandes für den Normalzustand oder den Übungszustand oder des zweiten lichtemittierenden Elements des Infrarot(IR)-Bandes für den Schlafzustand ausgibt und mindestens ein Lichtempfangselement der Vielzahl von Lichtempfangselementen aktiviert.

2. Elektronische Vorrichtung nach Anspruch 1, wobei eine Ausgangsintensität und/oder eine Ausgangswellenlänge des ersten lichtemittierenden Elements und des zweiten lichtemittierenden Elements unterschiedliche Attribute aufweisen.

3. Elektronische Vorrichtung nach Anspruch 1, wobei der mindestens eine Prozessor ferner zu Folgendem konfiguriert ist:
Steuern des ersten lichtemittierenden Elements und/oder des zweiten lichtemittierenden Elements, um Licht auszugeben, wenn eine Intensität des reflektierten Lichts, das dem Ausgangslicht entspricht, kleiner als oder gleich einem festgelegten Wert ist; und
Steuern des ersten lichtemittierenden Elements und des zweiten lichtemittierenden Elements, um Licht auszugeben, wenn die Intensität des reflektierten Lichts, das dem Ausgangslicht entspricht, größer als der festgelegte Wert ist.

4. Verfahren zum Erhalten biologischer Informationen einer elektronischen Vorrichtung (500), wobei die elektronische Vorrichtung (500) ein biometrisches Sensormodul umfasst, das ein lichtemittierendes Modul, das ein erstes lichtemittierendes Element (512a) mit einem ersten Attribut und ein zweites lichtemittierendes Element (512b) mit einem zweiten Attribut enthält, und ein Lichtempfangsmodul umfasst, das eine Vielzahl von Lichtempfangselementen (514a, 514b, 514c) enthält, die angrenzend an das lichtemittierende Modul angeordnet sind, wobei das Verfahren von einem Prozessor der besagten elektronischen Vorrichtung implementiert wird und Folgendes umfasst:
Bestimmen (620) eines Tragezustands basierend auf einer Distanz zwischen dem biometrischen Sensormodul und einem externen Objekt basierend auf zumindest reflektiertem Licht, das durch eine Kollision mit dem externen Objekt unter Lichtausgabe von dem ersten lichtemittierenden Element und dem zweiten lichtemittierenden Element des lichtemittierenden Moduls reflektiert wird;
Bestimmen (630) eines Bewegungszustands aus einem Normalzustand, einem Schlafzustand oder einem Übungszustand basierend auf Bewegungsinformationen von einem Bewegungssensor, der in der besagten elektronischen Vorrichtung enthalten ist;
Auswählen (640) des ersten lichtemittierenden Elements und/oder des zweiten lichtemittierenden Elements basierend auf dem Tragezustand und dem Bewegungszustand; und
Erhalten (650) biometrischer Informationen über das externe Objekt unter Verwendung des ausgewählten lichtemittierenden Elements,
wobei, wenn die Distanz kleiner als ein festgelegter Wert ist, das Verfahren ferner das Bestimmen des Tragezustands als eine erste enge Kontaktebene, die einem dichten Zustand entspricht, das Ausgeben von Licht unter Verwendung des ersten lichtemittierenden Elements und des zweiten lichtemittierenden Elements verschiedener Bänder und das Aktivieren nur eines Lichtempfangselements der Vielzahl von Lichtempfangselementen umfasst,
wobei, wenn die Distanz größer als oder gleich dem festgelegten Wert ist, das Verfahren ferner das Bestimmen des Tragezustands als eine zweite enge Kontakteben, die einem lockeren Zustand entspricht, das Auswählen des ersten lichtemittierenden Elements oder des zweiten lichtemittierenden Elements zum Ausgeben von Licht basierend auf dem bestimmten Bewegungszustand und das Ausgeben von Licht unter Verwendung des ausgewählten ersten lichtemittierenden Elements des grünen Bandes für den Normalzustand oder den Übungszustand oder des zweiten lichtemittierenden Elements des Infrarot(IR)-Bandes für den Schlafzustand und das Aktivieren mindestens eines Lichtempfangselements der Vielzahl von Lichtempfangselementen umfasst.

5. Verfahren nach Anspruch 4, wobei eine Ausgangsintensität und/oder eine Ausgangswellenlänge des ersten lichtemittierenden Elements und des zweiten lichtemittierenden Elements unterschiedliche Attribute aufweisen.

6. Verfahren nach Anspruch 4, das ferner Folgendes umfasst:
wenn eine Intensität des reflektierten Lichts, das dem Ausgangslicht entspricht, kleiner als oder gleich einem festgelegten Wert ist, Ausgeben von Licht unter Verwendung des ersten lichtemittierenden Elements und/oder des zweiten lichtemittierenden Elements; und
wenn die Intensität des reflektierten Lichts, das dem Ausgangslicht entspricht, größer als der festgelegte Wert ist, Ausgeben von Licht durch das erste lichtemittierende Element und das zweite lichtemittierende Element.

## Revendications

1. Dispositif électronique (500) comprenant :
un module de capteur biométrique (510) incluant :
un module d'émission de lumière comprenant un premier élément d'émission de lumière (512a) ayant un premier attribut et un deuxième élément d'émission de lumière (512b) ayant un deuxième attribut, et
un module de réception de lumière incluant une pluralité d'éléments de réception de lumière (514a, 514b, 514c) disposés adjacents au module d'émission de lumière ;
un capteur de mouvement configuré pour détecter un mouvement du dispositif électronique (500) ; et
au moins un processeur (530) configuré pour :
déterminer un état de port sur la base d'une distance entre le module de capteur biométrique et un objet externe sur la base d'au moins une lumière réfléchie réfléchie par collision avec l'objet externe parmi une émission de lumière du premier élément d'émission de lumière et du deuxième élément d'émission de lumière du module d'émission de lumière,
déterminer un état de mouvement de l'un parmi un état normal, un état de sommeil et un état d'exercice, sur la base d'informations de mouvement provenant du capteur de mouvement,
sélectionner au moins l'un parmi le premier élément d'émission de lumière ou le deuxième élément d'émission de lumière sur la base de l'état de port et de l'état de mouvement, et
obtenir des informations biométriques sur l'objet externe en utilisant l'élément d'émission de lumière sélectionné,
où si la distance est inférieure à une valeur désignée, le processeur détermine que l'état de port est un premier niveau de contact étroit correspondant à un état serré, émet de la lumière en utilisant le premier élément d'émission de lumière et le deuxième élément d'émission de lumière de diverses bandes et active un seul élément de réception de lumière de la pluralité d'éléments de réception de lumière,
où si la distance est supérieure ou égale à la valeur désignée, le processeur détermine que l'état de port est un deuxième niveau de contact étroit correspondant à un état desserré, sélectionne l'un parmi le premier élément d'émission de lumière et le deuxième élément d'émission de lumière pour émettre de la lumière sur la base de l'état de mouvement déterminé, émet de la lumière en utilisant ledit sélectionné parmi le premier élément d'émission de lumière de bande verte pour l'état normal ou l'état d'exercice et le deuxième élément d'émission de lumière de bande infrarouge, IR, pour l'état de sommeil et active au moins un élément de réception de lumière de la pluralité d'éléments de réception de lumière.

2. Dispositif électronique selon la revendication 1, où au moins une parmi une intensité émise ou une longueur d'onde émise du premier élément d'émission de lumière et du deuxième élément d'émission de lumière ont des attributs différents.

3. Dispositif électronique selon la revendication 1, où l'au moins un processeur est en outre configuré pour :
commander à au moins l'un parmi le premier élément d'émission de lumière et le deuxième élément d'émission de lumière d'émettre de la lumière lorsqu'une intensité de lumière réfléchie correspondant à la lumière émise est inférieure ou égale à une valeur désignée ; et
commander au premier élément d'émission de lumière et au deuxième élément d'émission de lumière d'émettre de la lumière lorsque l'intensité de la lumière réfléchie correspondant à la lumière émise est supérieure à la valeur désignée.

4. Procédé d'obtention d'informations biologiques d'un dispositif électronique (500), où le dispositif électronique (500) comprend un module de capteur biométrique comprenant un module d'émission de lumière incluant un premier élément d'émission de lumière (512a) ayant un premier attribut et un deuxième élément d'émission de lumière (512b) ayant un deuxième attribut, et un module de réception de lumière incluant une pluralité d'éléments de réception de lumière (514a, 514b, 514c) disposés adjacents au module d'émission de lumière, le procédé étant mis en œuvre par un processeur dudit dispositif électronique et comprenant :
déterminer (620) un état de port sur la base d'une distance entre le module de capteur biométrique et un objet externe sur la base d'au moins la lumière réfléchie réfléchie par collision avec l'objet externe parmi une émission de lumière du premier élément d'émission de lumière et du deuxième élément d'émission de lumière du module d'émission de lumière ;
déterminer (630) un état de mouvement de l'un parmi un état normal, un état de sommeil et un état d'exercice, sur la base d'informations de mouvement provenant d'un capteur de mouvement compris dans ledit dispositif électronique ;
sélectionner (640) au moins l'un parmi le premier élément d'émission de lumière et le deuxième élément d'émission de lumière sur la base de l'état de port et de l'état de mouvement ; et
obtenir (650) des informations biométriques sur l'objet externe en utilisant l'élément d'émission de lumière sélectionné,
où si la distance est inférieure à une valeur désignée, le procédé comprend en outre de déterminer l'état de port comme étant un premier niveau de contact étroit correspondant à un état serré, émettre de la lumière en utilisant le premier élément d'émission de lumière et le deuxième élément d'émission de lumière de diverses bandes et activer un seul élément de réception de lumière de la pluralité d'éléments de réception de lumière,
où si la distance est supérieure ou égale à la valeur désignée, le procédé comprend en outre de déterminer l'état de port comme étant un deuxième niveau de contact étroit correspondant à un état desserré, sélectionner l'un parmi le premier élément d'émission de lumière et le deuxième élément d'émission de lumière pour émettre de la lumière sur la base de l'état de mouvement déterminé, et émettre de la lumière en utilisant ledit sélectionné parmi le premier élément d'émission de lumière de bande verte pour l'état normal ou l'état d'exercice et le deuxième élément d'émission de lumière de bande infrarouge, IR, pour l'état de sommeil et activer au moins un élément de réception de lumière de la pluralité d'éléments de réception de lumière.

5. Procédé selon la revendication 4, où au moins une parmi une intensité émise ou une longueur d'onde émise du premier élément d'émission de lumière et du deuxième élément d'émission de lumière ont des attributs différents.

6. Procédé selon la revendication 4, comprenant en outre :
lorsqu'une intensité de lumière réfléchie correspondant à la lumière émise est inférieure ou égale à une valeur désignée, émettre de la lumière en utilisant au moins l'un parmi le premier élément d'émission de lumière et le deuxième élément d'émission de lumière ; et
lorsque l'intensité de la lumière réfléchie correspondant à la lumière émise est supérieure à la valeur désignée, émettre de la lumière par le premier élément d'émission de lumière et le deuxième élément d'émission de lumière.
